# FASCICULE DE BREVET EUROPEEN

(11) **EP 3 442 695 B1**
(45) Date de publication et mention de la délivrance du brevet: **17.07.2024**
(21) Numéro de dépôt: 17721785.8
(22) Date de dépôt: 14.04.2017
(51) Int. Cl.: B01J 8/18, C10G 2/00, B01J 8/00, C07C 29/151, C07C 31/04, C07C 1/12

(54) **DISPOSITIF ET PROCÉDÉ DE COGÉNÉRATION DE MÉTHANOL ET DE MÉTHANE DE SYNTHÈSE**
VORRICHTUNG UND VERFAHREN FÜR DIE GLEICHZEITIGE ERZEUGUNG VON METHANOL UND SYNTHETISCHEM METHAN
DEVICE AND METHOD FOR COGENERATION OF METHANOL AND SYNTHETIC METHANE

(30) Priorité: 15.04.2016 FR 1653354
(43) Date de publication de la demande: 20.02.2019
(73) Titulaire: ENGIE, 92400 Courbevoie (FR)
(72) Inventeur: KARA, Yilmaz, 95600 Eaubonne (FR); FORTIN, Stéphane, 59850 Nieppe (FR); NGUYEN, Jérôme, 92400 Courbevoie (FR); BARBA, Alessandra, 92400 Courbevoie (FR)
(74) Mandataire: Cornuejols, Georges
(86) Numéro de dépôt international: PCT/FR2017/050902
(87) Numéro de publication internationale: WO 2017/178771

(56) Documents cités:
- WO-A1-2005/108336
- WO-A2-2016/063043
- CN-A- 104 232 195
- US-A1- 2003 039 600
- US-A1- 2007 027 221
- US-A1- 2009 069 452
- WILLIAM L. LUYBEN: "Design and Control of Gas-Phase Reactor/Recycle Processes with Reversible Exothermic Reactions", INDUSTRIAL & ENGINEERING CHEMISTRY RESEARCH., vol. 39, no. 6, 1 June 2000 (2000-06-01), US, pages 1529 - 1538, XP055331602, ISSN: 0888-5885, DOI: 10.1021/ie990083w

## Description

### DOMAINE TECHNIQUE DE L'INVENTION

La présente invention vise un dispositif d'hydrogénation du CO₂ pour produire du méthanol, un procédé d'hydrogénation du CO₂ pour produire du méthanol, un dispositif de cogénération de méthanol et de méthane de synthèse et un procédé de cogénération de méthanol et de méthane de synthèse. Elle s'applique, notamment, au stockage d'énergie électrique intermittente sous forme d'un produit chimique stockable.

### ETAT DE LA TECHNIQUE

La diminution des ressources d'énergie fossile et la lutte contre le réchauffement climatique orientent l'industrie vers de nouveaux modes de production, afin d'élaborer des produits biosourcés ou pour stocker de l'énergie électrique renouvelable intermittente sous forme de produits facilement stockables tout en réutilisant du CO₂.

Ainsi, les modes de production de deux molécules sont particulièrement étudiées, le méthane et le méthanol qui tiennent une place clé dans les procédés de l'industrie chimique.

Fondamentalement, le méthanol et le méthane peuvent être synthétisés par des réactions d'hydrogénation du dioxyde de carbone (CO₂) ou du monoxyde de carbone (CO).

Du fait des équilibres thermochimiques de ces réactions catalytiques exothermiques, la conversion est globalement favorisée par une pression élevée et/ou une température maîtrisée. Par ailleurs, la présence de catalyseurs impose une maîtrise efficace des températures pour éviter la dégradation du catalyseur.

Les technologies de production de gaz de synthèse contenant CO, CO₂, et H₂ sont actuellement disponibles. Elles sont par exemple basées sur la gazéification ou l'électrolyse à haute température d'un mélange d'eau et de CO₂.

Les technologies basées sur l'électrolyse permettent de valoriser des surplus d'énergie renouvelable en gaz pouvant être transformé en méthanol (CH₃OH) ou en méthane de synthèse (dit « SNG », pour Synthesis Natural Gas, traduit en français par gaz naturel de synthèse).

La surproduction d'électricité étant généralement intermittente, l'enjeu des technologies actuelles est de proposer un procédé de valorisation capable de gérer ces fluctuations et cette intermittence. Par ailleurs, le marché et la demande en méthanol n'étant pas constant, le procédé doit également permettre une valorisation continue des surplus d'électricité en un vecteur facilement stockable et valorisable.

On trouve dans la littérature technique de nombreux procédés dédiés à la conversion thermochimique d'un gaz de synthèse en méthanol ou en méthane de synthèse. Traditionnellement les deux grandes familles de réacteurs sont : les réacteurs adiabatiques ou les réacteurs isothermes.

Dans les « réacteurs adiabatiques », la chaleur de réaction conduit à l'augmentation de la température du milieu réactionnel avec la conversion. Les principaux inconvénients de ces solutions sont :
- un fonctionnement multi-étage de réacteurs et de refroidissement,
- un fonctionnement à haute pression et
- un risque de dégradation du catalyseur par frittage (pics de température).

Les réacteurs isothermes se distinguent eux sur les techniques mises en oeuvre pour maîtriser l'exothermicité des réactions. Ces réacteurs sont généralement des « réacteurs échangeurs », des « réacteurs à eau bouillante » ou encore des « réacteurs à lit fluidisé ».

Synthèse du méthanol :
Le premier réacteur de synthèse « adiabatique » du méthanol a été élaboré par BASF en 1923. Il était exploité à 350°C et entre 250 - 350 bar. De nombreuses versions ont ensuite été proposées. Elles se distinguent principalement sur l'agencement des étages catalytiques, l'injection des réactifs ou les solutions de refroidissement entres étages catalytiques. On peut par exemple citer la technologie basse pression de ICI, le ARC Converter de Casai, le procédé Kellogg, ou encore le CMD (Collect Mix Distribute) de Haldor Topsoe.

En synthèse « isotherme », les développements sont également nombreux. On peut par exemple citer le « Tube Cooled Converter » de Johnson Mattey qui est un réacteur échangeur. La littérature technique autour du BWR fait par exemple référence au « Linde Isothermal Reactor », au « Steam Raising Converter » ou au « Radial Steam Raising Converter » de Johnson Matthey, ou au procédé MégaMéthanol de Lurgi. Casade SA a déposé une série de demandes de brevets (EP1350560, EP2070590, EP1600208). Enfin, les documents GB 2202531, US4956392, US5512599, US2007/0299146, US5216034 et US20070027220 présentent des procédés à lit fluidisé.

Parmi les inventions mentionnées précédemment, aucune solution de flexibilisation des quantités de CH₃OH produit et/ou de co-valorisation des excès de réactifs ne sont décrits.

Synthèse du méthane (SNG) :
Face à des verrous technologiques très proches, les technologies de production du méthane de synthèse sont généralement très inspirées des procédés de production de méthanol CH₃OH et vis-et-versa. Ainsi, les approches technologiques décrites pour la production de méthanol sont également possibles pour la production de méthane de synthèse (SNG).

Dans le cas des réacteurs spécifiquement conçus pour la méthanation, on peut par exemple citer le TWR (Throughwall Cooled Reactor) et les demandes de brevets US 2662911, US 2740803, US4636365, US6958153 ou US4339413.

Le système de réacteur BWR a également récemment fait l'objet d'adaptation pour la méthanation du CO₂ et est probablement applicable à la méthanation d'un syngas de gazéification ou de co-électrolyse. Les solutions actuellement proposées en lit fluidisé se distinguent entres-elles sur la manière d'assurer le refroidissement du réacteur. Parmi celles-ci, la littérature technique décrit le réacteur COMFLUX de Thyssen. Des tubes échangeurs, disposés verticalement dans le réacteur et suspendu au ciel de la zone de désengagement assure l'évacuation de la chaleur (US4539016). Le refroidissement est assuré par l'ébullition d'un liquide, lequel peut être de l'eau.

L'installation pilote mise en oeuvre à Güssing par le Paul Scherrer Institut (PSI) (EP1568674A1, WO2009/007061A1) mettait en oeuvre un système de refroidissement constitué, de façon similaire au dispositif retenu par COMFLUX, par un faisceau de tubes disposés verticalement dans le lit (Schildhauer T., 2010).

Co-synthèse ou Cogénération de méthanol et de méthane de synthèse (SNG) :
Concernant la cogénération de méthanol et de méthane de synthèse, la demande de brevet chinois CN1 04232195A / 2014 fait mention d'une méthode de production de méthanol et de méthane de synthèse liquéfié à partir de gaz de coke (H₂, CH₄, CO). Le gaz de coke est mélangé après différentes étapes d'épuration avec du CO₂ contenu dans les fumées de combustion et préalablement séparé par absorption aux amines. Une première étape réactionnelle consiste à faire réagir le syngas H₂, CO et CO₂ pour produire CH₃OH. L'excès de syngas est ensuite converti en méthane de synthèse au travers de deux ou trois réacteurs positionnés en série.

Le document US2003039600 décrit un schéma de contrôle pour la conversion du gaz de synthèse de la composition variable en carburant liquide dans un réacteur à colonne à bulles à trois phases ou à lames (SBCR). Le schéma de contrôle permet d'obtenir une production de carburant liquide constante et un flux de gaz de purge constant ou limité avec une condition d'alimentation en gaz de synthèse très variable. Ceci est réalisé en ajustant une ou plusieurs des variables indépendantes suivantes : rapport de recyclage, ajout d'eau et flux de dérivation.

Le document WO2005108336 décrit un système et un procédé pour la synthèse du méthanol à partir d'un gaz source contenant des hydrocarbures légers. Le système comprend : une alimentation de gaz d'hydrocarbure pour fournir le gaz source ; un apport en oxygène pour fournir un fluide contenant de l'oxygène; un réacteur de gaz de synthèse sous la forme d'un réacteur de gazéification, prévu pour recevoir le gaz de source provenant de l'alimentation en hydrocarbure et le fluide contenant de l'oxygène provenant de l'alimentation en oxygène ; un réacteur de méthanol agencé pour recevoir au moins une fraction du produit de réaction intermédiaire du réacteur de gaz de synthèse, et agencé pour laisser réagir le produit de réaction intermédiaire dans au moins du méthanol.

Le document US2009069452 décrit un procédé de conversion de gaz de synthèse en éthanol et / ou d'autres alcools supérieurs. Des modes de réalisation préférés recyclent le méthanol, le convertissent partiellement en gaz de synthèse, puis convertissent ces gaz de synthèse supplémentaires également en C₂ + alcools. Généralement, il est prévu des réacteurs comprenant des catalyseurs capables de convertir du gaz de synthèse en alcools avec de faibles sélectivités pour le dioxyde de carbone et le méthane, et des stratégies de procédé pour séparer et recycler le gaz de synthèse n'ayant pas réagi ainsi que le méthanol produit par le catalyseur.

Le document US2007027221 décrit un procédé de fabrication d'un produit de méthanol en utilisant un réacteur à bulles de boue. Le réacteur fonctionne en phase liquide, les particules de catalyseur étant en suspension dans le liquide. Il est prévu une utilisation de la chaleur et la récupération du produit en alimentant du gaz de synthèse frais dans le réacteur, tout en maintenant un degré élevé de rétro-mélange dans le réacteur.

Le document William L. Luyben « Design and control of Gas-phase reactor/recycle processes with réversibles Exothermic Reactions" (Industrial & Engineering Chemistry Resarch, vol. 39, no. 6, pages 1529-1538) considère les aspects de design et de contrôle d'un système ternaire avec une réaction en phase gazeuse réversible, exothermique dans un réacteur adiabatique tubulaire comportant un catalyseur solide.

Aucun des systèmes mentionnés ci-dessus ne permet :
- de valoriser les pics de surproduction électrique d'origine renouvelable,
- de réduire et valoriser les émissions de gaz à effets de serre comme le CO₂,
- de produire un méthanol à haute valeur ajoutée aussi bien corrélée à la disponibilité de l'électricité renouvelable qu'à la demande du marché pour ce composé et
- de stocker l'excès de réactif sous forme d'un méthane de synthèse directement injectable dans un réseau de gaz naturel.

### OBJET DE L'INVENTION

La présente invention vise à remédier à tout ou partie de ces inconvénients. Les caractéristiques essentielles de l'invention sont explicitement définies dans les libellés des revendications 1 et 9. D'autres caractéristiques de l'invention sont définis dans les libellés des revendications dépendantes.

A cet effet, selon un premier aspect, la présente invention vise un dispositif de cogénération de méthanol et de méthane de synthèse comportant :
- un dispositif d'hydrogénation du CO₂ pour produire du méthanol à partir d'un syngas comportant :
   - du dihydrogène, H₂, sorti par exemple d'un dispositif d'électrolyse de l'eau, H₂O, et
   - du dioxyde de carbone, CO₂,
   ledit dispositif d'hydrogénation comportant :
   - un réacteur d'hydrogénation du CO₂ pour produire du méthanol, dite « hydrogénation », comportant :
      - une entrée pour le syngas et
      - une sortie pour des produits de la réaction d'hydrogénation comportant au moins du méthanol, CH₃OH ou MeOH,
   - une première conduite de recirculation, d'au moins une partie des produits chauds de la réaction d'hydrogénation reliant la sortie du réacteur à l'entrée du réacteur, comportant un recirculateur, dit « premier recirculateur », de ladite partie des produits de réaction,
   - un moyen de mesure du débit de syngas traversant l'entrée du réacteur positionné en amont d'une jonction entre la conduite de recirculation et l'entrée du réacteur et
   - un moyen de commande du premier recirculateur configuré pour commander la recirculation d'une quantité de produits chauds de réaction d'hydrogénation déterminée en fonction du débit de syngas mesuré et d'une première valeur consigne prédéterminée et
- un réacteur de méthanation comportant :
   - une entrée pour une partie des produits d'hydrogénation comportant au moins du syngas en excès suite à la réaction d'hydrogénation et
- une sortie pour des produits de réaction de méthanation comportant au moins du méthane de synthèse.

Grâce à ces dispositions, le dispositif garantit un débit traversant le réacteur constant par rapport aux hypothèses de conception. Le gaz recirculé étant à la température de réaction, aucun effet thermique n'est à prévoir par l'alimentation de ce gaz au réacteur. Ces dispositions confèrent à ce dispositif une très grande flexibilité de fonctionnement, propriété très importante pour un fonctionnement en adéquation avec l'intermittence des systèmes de production d'électricité renouvelable.

Ces dispositions permettent à la fois une valorisation de l'excès d'énergie électrique en méthanol et, lorsque la demande en méthanol n'est pas suffisante, en méthane de synthèse stockable dans les réseaux de gaz naturel.

Le dispositif d'hydrogénation objet de la présente invention comporte un condenseur, des produits de réaction d'hydrogénation sortis du réacteur, positionné en aval d'une jonction entre la conduite de recirculation et la sortie du réacteur, pour séparer au moins le méthanol et l'eau du syngas en excès suite à la réaction d'hydrogénation.

Ces modes de réalisation permettent de séparer le syngas en excès du méthanol et de l'eau, ce méthanol pouvant être stocké ou fourni, après séparation partielle ou totale de l'eau par exemple au travers d'une colonne de distillation, à un dispositif externe dont le fonctionnement nécessite du méthanol.

Le dispositif d'hydrogénation objet de la présente invention comporte :
- une première canalisation de recirculation, du syngas froid en excès en sortie du condenseur, reliant la sortie du condenseur et l'entrée du réacteur d'hydrogénation, en amont du point de mesure du débit de syngas par le moyen de mesure, comportant un recirculateur, dit « deuxième recirculateur », dudit syngas,
- un moyen de mesure du débit du méthanol condensé par le condenseur et
- un moyen de commande du deuxième recirculateur configuré pour commander la recirculation d'une quantité de produits en excès de la réaction d'hydrogénation déterminée en fonction du débit de méthanol condensé mesuré et d'une deuxième valeur consigne prédéterminée.

Ces modes de réalisation permettent d'ajuster le débit de production de méthanol en fonction d'une demande déterminée en aval du dispositif objet de la présente invention. Ainsi, le dispositif présente une double flexibilité : l'une relative à la quantité de H₂ disponible en entrée et l'autre relative à la demande de MeOH en sortie.

Dans des modes de réalisation, le dispositif objet de la présente invention comporte un capteur de température de réaction dans le, ou en sortie du, réacteur d'hydrogénation, l'entrée pour syngas du réacteur comportant un échangeur de chaleur dont la température de sortie est déterminée en fonction de la température de réaction captée.

Ces modes de réalisation permettent d'assurer l'isothermicité du réacteur d'hydrogénation.

Dans des modes de réalisation, le dispositif objet de la présente invention comporte :
- une deuxième conduite de recirculation, d'au moins une partie des produits chauds de la réaction de méthanation reliant la sortie du réacteur de méthanation à l'entrée du réacteur de méthanation, comportant un recirculateur, dit « troisième recirculateur », de ladite partie des produits,
- un moyen de mesure du débit de syngas traversant l'entrée du réacteur de méthanation positionné en amont d'une jonction entre la deuxième conduite de recirculation et l'entrée du réacteur de méthanation et
- un moyen de commande du troisième recirculateur configuré pour commander la recirculation d'une quantité de produits chauds de réaction de méthanation déterminée en fonction du débit de syngas mesuré et d'une troisième valeur consigne prédéterminée.

Ces modes de réalisation permettent de conférer au réacteur de méthanation une importante flexibilité au regard du débit de syngas entrant. Ainsi, quel que soit la part d'énergie électrique, convertie en H₂, puis en MeOH, la part restante de syngas en excès peut être convertie en méthane de synthèse.

Dans des modes de réalisation, le dispositif objet de la présente invention comporte un séparateur d'eau contenue dans les produits de méthanation.

Ces modes de réalisation permettent de retirer l'eau du flux de méthane de synthèse de manière à répondre aux spécifications d'injection de méthane sur un réseau gazier.

Dans des modes de réalisation, le dispositif objet de la présente invention comporte :
- une deuxième canalisation de recirculation, du méthane de synthèse froid en sortie du séparateur, reliant la sortie du séparateur et l'entrée du réacteur de méthanation, en amont du point de mesure du débit de syngas par le moyen de mesure, comportant un recirculateur, dit « quatrième recirculateur », dudit méthane de synthèse,
- un moyen de mesure de la température de réaction de méthanation à l'intérieur, ou en sortie, du réacteur de méthanation et
- un moyen de commande du quatrième recirculateur configuré pour commander la recirculation d'une quantité de méthane de synthèse déterminée en fonction de la température mesurée.

Ces modes de réalisation permettent le refroidissement du réacteur de méthanation par volant thermique et le maintien isotherme du réacteur de méthanation.

Dans des modes de réalisation, le dispositif objet de la présente invention comporte :
- un moyen de mesure d'une caractéristique de composition du méthane de synthèse sorti du réacteur de méthanation et
- un moyen de régulation d'un débit de CO₂ injecté dans l'entrée pour syngas du réacteur d'hydrogénation, ce moyen de régulation étant commandé en fonction de la caractéristique mesurée et d'une quatrième valeur prédéterminée.

Ces modes de réalisation permettent de limiter les excès de H₂ et/ou de CO₂ dans les produits de la réaction de méthanation et d'ajuster le pouvoir calorifique supérieur, dit « PCS », ou l'indice de Wobbe du méthane de synthèse avant injection sur un réseau gazier. Cette régulation permet, de plus, d'éviter d'avoir à réaliser des séparations coûteuses et complexes du CO₂ et/ou du H₂ pour atteindre ces spécifications.

Dans des modes de réalisation, le dispositif objet de la présente invention comporte un détendeur du syngas entre le dispositif d'hydrogénation et l'entrée du réacteur de méthanation ou en aval du réacteur de méthanation.

Ces modes de réalisation permettent d'adapter la pression de fonctionnement de l'étape de méthanation à la pression de conditionnement et d'utilisation du méthane de synthèse.

Dans des modes de réalisation, le dispositif objet de la présente invention comporte en amont du détendeur, un échangeur de chaleur dont la température de sortie est déterminée en fonction d'une température du syngas mesurée, par un moyen de mesure de température, en aval du détendeur.

Ces modes de réalisation permettent d'éviter une condensation partielle du CO₂ après détente du syngas à la pression d'injection du méthane de synthèse produit. En effet, l'effet joule de la détente peut conduire à un sur refroidissement du syngas pouvant conduire à une condensation partielle du CO₂ si la température en amont est maintenue à la température de sortie du séparateur.

Selon un deuxième aspect, la présente invention vise un procédé de cogénération de méthanol et de méthane de synthèse, qui comporte :
- un procédé d'hydrogénation du CO₂ pour produire du méthanol pour produire du méthanol à partir d'un syngas comportant :
   - du dihydrogène, H₂, sorti par exemple d'une étape d'électrolyse de l'eau, H₂O, et
   - du dioxyde de carbone, CO₂,
   ledit procédé d'hydrogénation comportant :
   - une étape de réaction d'hydrogénation du CO₂ pour produire du méthanol, dite « hydrogénation », dans un réacteur d'hydrogénation, comportant :
      - une étape d'entrée pour le syngas et
      - une étape de sortie pour des produits de la réaction d'hydrogénation comportant au moins du méthanol, CH₃OH ou MeOH,
   - une étape de mesure du débit de syngas traversant l'entrée du réacteur en amont d'une jonction entre une conduite de recirculation, reliant la sortie du réacteur à l'entrée du réacteur, dans une conduite de canalisation comportant un recirculateur, dit « premier recirculateur », de ladite partie des produits,
   - une étape de commande du premier recirculateur configuré pour commander la recirculation d'une quantité de produits de réaction d'hydrogénation déterminée en fonction du débit de syngas mesuré et d'une première valeur consigne prédéterminée et
   - une première étape de recirculation, d'au moins une partie des produits chauds de la réaction d'hydrogénation dans la conduite de recirculation et
- une étape de réaction de méthanation, réalisée par un réacteur de méthanation, comportant :
   - une étape d'entrée pour une partie des produits d'hydrogénation comportant au moins du syngas en excès suite à la réaction d'hydrogénation et
   - une étape de sortie pour des produits de réaction de méthanation comportant au moins du méthane de synthèse.

Dans des modes de réalisation, le procédé objet de la présente invention comporte :
- une étape de mesure du débit de syngas traversant l'entrée du réacteur de méthanation positionné en amont d'une jonction entre une deuxième conduite de recirculation reliant la sortie du réacteur de méthanation à l'entrée du réacteur de méthanation, comportant un recirculateur, dit « troisième recirculateur »,
- une étape de commande du troisième recirculateur configuré pour commander la recirculation d'une quantité de produits de réaction de méthanation déterminée en fonction du débit de syngas mesuré et d'une troisième valeur consigne prédéterminée et
- une deuxième étape de recirculation, d'au moins une partie des produits chauds de la réaction de méthanation dans la deuxième conduite de recirculation.

Les buts, avantages et caractéristiques particulières du procédé objet de la présente invention étant similaires à ceux du dispositif de cogénération objet de la présente invention, ils ne sont pas rappelés ici.

### BREVE DESCRIPTION DES FIGURES

D'autres avantages, buts et caractéristiques particulières de l'invention ressortiront de la description non limitative qui suit d'au moins un mode de réalisation particulier du dispositif et du procédé objets de la présente invention, en regard des dessins annexés, dans lesquels :
- la figure 1 représente, schématiquement, un premier mode de réalisation particulier du dispositif objet de la présente invention,
- la figure 2 représente, schématiquement et sous forme d'un logigramme, une succession d'étapes particulière du procédé d'hydrogénation du CO₂ pour produire du méthanol objet de la présente invention,
- la figure 3 représente, schématiquement et sous forme d'un logigramme, une succession d'étapes particulière du procédé de cogénération objet de la présente invention,
- la figure 4 représente, sous forme d'un graphe, un exemple de la fraction molaire de carbone convertie en CH₃OH ou en CH₄ en fonction d'un ratio de recirculation obtenue par la mise en oeuvre du dispositif de cogénération objet de la présente invention,
- la figure 5 représente, sous forme d'un graphe, un exemple de la fraction molaire de carbone convertie en CH₃OH ou en CH₄ en fonction de la température du réacteur d'hydrogénation obtenue par la mise en oeuvre du dispositif de cogénération objet de la présente invention,
- la figure 6 représente, sous forme d'un graphe, un exemple de l'indice de Wobbe et du PCS en fonction d'un ratio de recirculation obtenus par la mise en oeuvre du dispositif de cogénération objet de la présente invention,
- la figure 7 représente, sous forme d'un graphe, un exemple du ratio H₂/CO₂ en fonction d'une température du réacteur d'hydrogénation obtenue par la mise en oeuvre du dispositif d'hydrogénation objet de la présente invention,
- la figure 8 représente, sous forme d'un graphe, un exemple du ratio H₂/CO₂ en fonction d'un taux de recirculation obtenue par la mise en oeuvre du dispositif d'hydrogénation objet de la présente invention,
- la figure 9 représente, sous forme d'un graphe, un exemple de la température en sortie d'échangeur, en amont de la réaction d'hydrogénation, en fonction de la température du réacteur d'hydrogénation obtenue par la mise en oeuvre du dispositif d'hydrogénation objet de la présente invention et
- la figure 10 représente, sous forme d'un graphe, un exemple de la température en sortie d'échangeur, en amont de la réaction d'hydrogénation, en fonction du taux de recirculation obtenue par la mise en oeuvre du dispositif d'hydrogénation objet de la présente invention.

### DESCRIPTION D'EXEMPLES DE REALISATION DE L'INVENTION

La présente description est donnée à titre non limitatif, chaque caractéristique d'un mode de réalisation pouvant être combinée à toute autre caractéristique de tout autre mode de réalisation de manière avantageuse. Par ailleurs, chaque paramètre d'un exemple de réalisation peut être mis en oeuvre indépendamment d'autres paramètres dudit exemple de réalisation.

On note dès à présent que les figures ne sont pas à l'échelle.

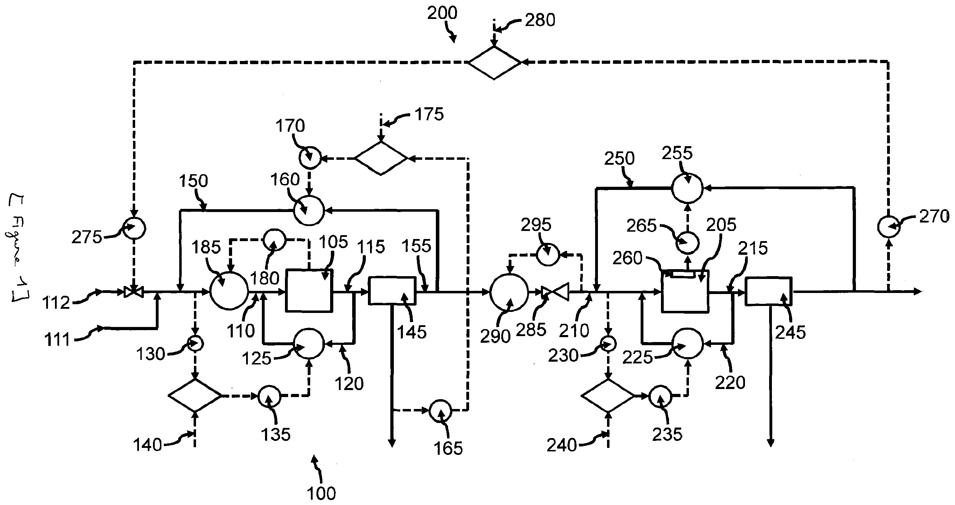

On observe, sur la figure 1, une vue schématique d'un mode de réalisation du dispositif 100 objet de la présente invention. Le dispositif 100 d'hydrogénation du CO₂ pour produire du méthanol à partir d'un syngas comportant :
- du dihydrogène 111, H₂, sorti par exemple d'un dispositif d'électrolyse de l'eau, H₂O, et
- du dioxyde de carbone 112, CO₂,
ledit dispositif 100 comportant :
- un réacteur 105 d'hydrogénation du CO₂ pour produire du méthanol, dite « hydrogénation », comportant :
   - une entrée 110 pour le syngas et
   - une sortie 115 pour des produits de la réaction d'hydrogénation comportant au moins du méthanol, CH₃OH ou MeOH,
- une première conduite 120 de recirculation, d'au moins une partie des produits chauds de la réaction d'hydrogénation reliant la sortie 115 du réacteur à l'entrée 110 du réacteur, comportant un recirculateur 125, dit « premier recirculateur », de ladite partie des produits chauds de réaction d'hydrogénation,
- un moyen 130 de mesure du débit de syngas traversant l'entrée du réacteur positionné en amont d'une jonction entre la conduite de recirculation et l'entrée du réacteur et
- un moyen 135 de commande du premier recirculateur configuré pour commander la recirculation d'une quantité de produits chauds de réaction d'hydrogénation déterminée en fonction du débit de syngas mesuré et d'une première valeur 140 consigne prédéterminée.

Le dispositif 100 comporte, en amont de l'entrée 110 pour syngas, une entrée 111 pour dihydrogène et une entrée 112 pour dioxyde de carbone. Chacune de ces entrées, 111 et 112, est positionnée en aval d'unités de production, de purification et de conditionnement (non représentées). Les flux issus de ces entrées, 111 et 112, sont mélangés pour former le syngas agissant comme réactif dans la réaction d'hydrogénation.

Le débit de syngas dépend de l'électricité disponible en amont du dispositif 100, cette électricité étant convertie en dihydrogène par exemple par électrolyse de l'eau.

Le réacteur 105 est un réacteur à lit fluidisé ou à lit fixe dépourvu en partie ou totalement de tubes de refroidissement interne. Ce réacteur 105 est configuré pour réaliser une réaction d'hydrogénation convertissant le syngas en méthanol et en eau.

La réaction catalytique d'hydrogénation est connue pour être globalement exothermique lors de la production de méthanol. Cette réaction est favorisée dans une plage de température comprise entre 200 et 320°C et des pressions élevées généralement supérieures à 70 bars. Les technologies de réacteur généralement utilisées sont classifiées selon deux familles :
- les réacteurs adiabatiques nécessitant plusieurs étapes couplées en série avec des refroidissements intermédiaires pour atteindre une hydrogénation satisfaisante et
- les réacteurs isothermes permettant d'atteindre une hydrogénation satisfaisante en une seule étape moyennant une extraction de la chaleur de réaction intégrée au réacteur 105 par le biais de surfaces d'échange.

Le réacteur 105 mis en oeuvre par le présent mode de réalisation peut être classifié parmi les réacteurs « isothermes ». Par contre, contrairement à ce qui est présenté dans l'art antérieur, le refroidissement ne nécessite pas l'installation de surfaces immergées ou nécessite juste une installation partielle pour assurer l'échange thermique avec la couche catalytique.

La sortie 115 du réacteur 105 transporte principalement de l'eau et du méthanol ainsi que du syngas en excès. L'excès de CO₂ et H₂ est conséquente à l'équilibre de la réaction d'hydrogénation qui dépend des conditions opératoires de températures et pressions.

Les variations de débit de syngas, en raison de paramètres de production inconstants, peuvent avoir un impact significatif sur le régime hydrodynamique et par conséquent thermique du réacteur 105 d'hydrogénation.

Pour pallier ces fluctuations, l'utilisation d'un réacteur suffisamment flexible en débit est indispensable. Selon l'art antérieur, cette flexibilité passe souvent par la conception d'un réseau de réacteurs disposés en parallèle avec une gestion très complexe des flux et du nombre de réacteurs à faire fonctionner et coexister. Dans le cas des technologies type lits fluidisés, la gamme « naturelle » de flexibilité est généralement plus large mais limité à un domaine compris entre 0,5 et 6 fois le débit nominal de construction.

La présente invention vise, pour cet objectif, un ensemble formé de la conduite de recirculation 120, du recirculateur 125, du moyen de mesure 130 de débit de syngas en entrée 110 du réacteur 105 et du moyen de commande 135 du recirculateur 125. Cet ensemble présente une flexibilité dans un domaine beaucoup plus large avec un débit quasi-constant et donc une forte stabilité de conversion au réacteur 105 d'hydrogénation.

La première conduite 120 de recirculation est, par exemple, une dérivation de tout ou partie du flux sorti du réacteur 105 vers l'entrée 110 du réacteur 105. Cette première conduite 120 comporte un recirculateur 125, ce recirculateur 125 étant, par exemple :
- un surpresseur mécanique comportant un variateur de vitesse ou
- un ensemble comportant un surpresseur mécanique à vitesse constante et un système de régulation de débit.

Le fait de recirculer un fluide de recirculation chaud avec une composition équivalente à la composition au sein du réacteur 105 à travers le réacteur 105 d'hydrogénation n'a aucune incidence sur les équilibres thermique et thermochimique de cette réaction mais permet de maintenir un débit hydrodynamique stable.

Cette recirculation est régulée par une boucle de régulation comportant le moyen de mesure 130 de débit de syngas en entrée 110 du réacteur 105 et le moyen de commande 135 du recirculateur 125.

Le moyen de mesure 130 est, par exemple, un débitmètre configuré pour réaliser une mesure du débit volumique de syngas traversant l'entrée 110 en amont de la recirculation des produits chauds de réaction.

Le moyen de commande 135 est, par exemple, un circuit électronique de commande configuré pour comparer le débit mesuré et une première valeur prédéterminée, correspondant au débit nominal de conception du réacteur 105 d'hydrogénation.

En fonction du décalage détecté, le moyen de commande 135 commande la recirculation de tout ou partie du flux de produits chauds de la réaction d'hydrogénation dans le réacteur 105 pour que le débit en entrée 110 de ce réacteur 105 soit proche du débit nominal de conception de ce réacteur 105.

Dans des modes de réalisation préférentiels, tel que celui représenté en figure 1, le dispositif 100 comporte un condenseur 145, des produits de réaction d'hydrogénation sortis du réacteur 105, positionné en aval d'une jonction entre la conduite 120 de recirculation et la sortie 115 du réacteur, pour séparer au moins le méthanol et l'eau du syngas en excès suite à la réaction d'hydrogénation.

Ce condenseur 145 est, par exemple, un condenseur par refroidissement configuré pour agir à une température permettant la séparation par condensation (-5°C à 60°C) du méthanol et de l'eau des réactifs incondensables en excès. Préférentiellement, la température du flux sortant du condenseur 145 est inférieure ou égale à la température de rosée du méthanol et de l'eau dans les conditions opératoires du réacteur d'hydrogénation. L'énergie récupérée dans le condenseur 145 par refroidissement des produits de réaction d'hydrogénation correspond à la chaleur des réactions de conversion dans le réacteur 105 d'hydrogénation, complétée des chaleurs latentes de changement de phase vapeur/gaz (vaporisation/condensation) du méthanol et de l'eau. Le condenseur 145 peut être par exemple un équipement unique de condensation (échangeur ou trempe par injection d'eau) ou un ensemble d'échangeurs en série ou parallèle permettant la récupération de chaleur à différentes températures.

Dans des modes de réalisation préférentiels, tel que celui représenté en figure 1, le dispositif 100 comporte :
- une première canalisation 150 de recirculation, du syngas froid en excès en sortie du condenseur 145, reliant la sortie 155 du condenseur et l'entrée 110 du réacteur 105 d'hydrogénation, en amont du point de mesure du débit de syngas par le moyen 130 de mesure, comportant un recirculateur 160, dit « deuxième recirculateur », dudit syngas,
- un moyen 165 de mesure du débit du méthanol condensé par le condenseur 145 et
- un moyen 170 de commande du deuxième recirculateur configuré pour commander la recirculation d'une quantité de produits en excès de réaction d'hydrogénation déterminée en fonction du débit de méthanol condensé mesuré et d'une deuxième valeur 175 consigne prédéterminée.

La première canalisation 150 est, par exemple, une dérivation de tout ou partie du flux incondensable sorti du condenseur 145 vers l'entrée 110 du réacteur 105. Cette première canalisation 150 comporte un recirculateur 160, ce recirculateur 160 étant, par exemple :
- un surpresseur mécanique comportant un variateur de vitesse ou
- un ensemble comportant un surpresseur mécanique à vitesse constante et un système de régulation de débit.

Ainsi, en aval de la jonction entre l'entrée 110 de syngas et la première canalisation 150 de recirculation, le syngas est complété par un flux correspondant à la recirculation d'une partie des réactifs incondensables récupérés en sortie du condenseur 145, l'autre sortie du condenseur 145 contenant principalement du méthanol et de l'eau en phase liquide.

Le moyen de mesure 165 du débit de méthanol condensé par le condenseur 145 est, par exemple, un débitmètre configuré pour réaliser une mesure du débit volumique de méthanol et d'eau sortis du condenseur 145 et une chromatographie en phase liquide pour mesurer la concentration volumique en méthanol de ces produits condensés. Dans ce cas, le débit de méthanol est déterminé par le produit entre le débit volumique de méthanol et d'eau sortis du condenseur 145 et la concentration volumique de méthanol. Cette mesure peut également être réalisée par une mesure simple du débit volumique de méthanol après une séparation (non représentée) du méthanol et de l'eau par exemple au travers d'une colonne de distillation.

Le moyen de commande 170 est, par exemple, un circuit électronique de commande configuré pour comparer le débit mesuré et une deuxième valeur prédéterminée, correspondant à une consommation de méthanol mesurée en aval du dispositif 100 ou à une consigne de production de méthanol.

Ainsi, au gré de la demande en méthanol, le dispositif 100 permet d'ajuster le débit de production de ce composé à plus haute valeur à un débit correspondant à cette demande. Le syngas en excès pouvant être utilisé dans d'autres applications et, en particulier, dans la génération de méthane de synthèse telle que décrite ci-dessous. L'autre fonction du recirculateur 160 est de compenser les pertes de charge (pertes de pression) principalement générées par l'échangeur 185, le réacteur 105 d'hydrogénation et le condenseur 145. De plus, la recirculation de syngas froid dans le réacteur 105 permet d'assurer l'isothermicité de ce réacteur 105 par simple « volant thermique » du flux de recirculation froid.

Dans des modes de réalisation préférentiels, tel que celui représenté en figure 1, le dispositif 100 comporte un capteur 180 de température de réaction dans le, ou en sortie du, réacteur 105 d'hydrogénation, l'entrée 110 pour syngas du réacteur comportant un échangeur 185 de chaleur dont la température de sortie est déterminée en fonction de la température de réaction captée.

La recirculation de syngas froid en entrée du réacteur 105 d'hydrogénation peut conduire à un déséquilibre thermique du réacteur par sur ou sous refroidissement en fonction de la température opératoire du réacteur 105 d'hydrogénation. Afin de dissocier les fonctions de flexibilité de production de méthanol et de maintien de l'isothermicité de réaction, le flux de syngas froid recirculé résultant du mélange traverse, dans ces modes de réalisation, l'échangeur 185 dont la température de sortie est régulée de sorte à obtenir une température de réaction d'hydrogénation constante.

Le capteur 180 est, par exemple, une sonde de température positionnée dans la sortie 115 ou dans le réacteur 105.

On observe également en figure 1, un mode de réalisation particulier du dispositif 200 objet de la présente invention. Ce dispositif 200 de cogénération de méthanol et de méthane de synthèse, comporte :
- un dispositif 100 d'hydrogénation tel que décrit ci-dessus,
- un réacteur 205 de méthanation comportant :
   - une entrée 210 pour une partie des produits d'hydrogénation comportant au moins du syngas en excès suite à la réaction d'hydrogénation et
   - une sortie 215 pour des produits de réaction de méthanation comportant au moins du méthane de synthèse.

Dans ce dispositif 200, l'excès de syngas en sortie du condenseur 145 est d'une part recirculé dans la première canalisation 150 de recirculation en fonction de la demande en méthanol et d'autre part destiné à la co-production de méthane de synthèse dans le dispositif 200.

Dans les procédés existants de production de méthanol, l'excès de syngas est généralement recyclé en amont du procédé pour constituer une partie des réactifs servants à la production de méthanol ou simplement purgé par élimination connexe. Le choix de la recirculation de cet excès donne lieu à une production constante qui n'offre pas à la technologie de caractère flexible et ne permet pas son adaptation au marché. Le dispositif 200 permet ici de valoriser l'excès comme syngas pour la production de méthane de synthèse.

Le réacteur 205 est, par exemple, un réacteur de méthanation à lit fixe, à lit refroidi par les parois, à eau bouillante ou de préférence à lit fluidisé.

Les surfaces d'échange de ce réacteur 205 peuvent être partiellement ou totalement supprimées.

Dans des modes de réalisation préférentiels, tel que celui représenté en figure 1, le dispositif 200 comporte :
- une deuxième conduite 220 de recirculation, d'au moins une partie des produits chauds de la réaction de méthanation reliant la sortie du réacteur de méthanation à l'entrée du réacteur de méthanation, comportant un recirculateur 225, dit « troisième recirculateur », de ladite partie des produits chauds,
- un moyen 230 de mesure du débit de syngas traversant l'entrée 210 du réacteur 205 de méthanation positionné en amont d'une jonction entre la deuxième conduite de recirculation et l'entrée du réacteur de méthanation et
- un moyen 235 de commande du troisième recirculateur configuré pour commander la recirculation d'une quantité de produits chauds de réaction de méthanation déterminée en fonction du débit de syngas mesuré et d'une troisième valeur 240 consigne prédéterminée.

Le débit du syngas est entièrement fonction des quantités résiduelles de H₂ et CO₂ consécutive à l'étape amont de production de méthanol, cette étape étant fonction de la demande en méthanol. Afin de conserver la stabilité de conversion au cours de l'opération de méthanation, il est préférable de maintenir des conditions hydrodynamiques les plus constantes possibles. Néanmoins, si la demande en méthanol est importante ou si l'électricité disponible pour la production de H₂ est trop faible, le débit de syngas à méthaner peut être drastiquement diminué. Pour ces cas de fonctionnement, il convient tout de même de maintenir un débit global entrant dans le réacteur 205 constant ou d'opter pour une technologie très flexible. Même dans le cas du lit fluidisé capable de fonctionner aisément dans une gamme de 0,5 à 6, des débits trop faibles peuvent engendrer une dégradation du refroidissement et donc de la conversion. Pour pallier cette difficulté en cas de baisse drastique de syngas, le flux de syngas est complété par une recirculation chaude provenant directement de la sortie 215 du réacteur 205 par le biais de la deuxième conduite 220 de recirculation. Le fait d'utiliser un fluide de recirculation chaud ne provoque pas de déséquilibre thermique du réacteur 205 mais permet de rendre le dispositif 200 très flexible.

La deuxième conduite 220 de recirculation est, par exemple, une dérivation de tout ou partie du flux sorti du réacteur 205 vers l'entrée 210 du réacteur 205. Cette deuxième conduite 220 comporte un recirculateur 225, ce recirculateur 225 étant, par exemple :
- un surpresseur mécanique comportant un variateur de vitesse ou
- un ensemble comportant un surpresseur mécanique à vitesse constante et un système de régulation de débit.

Le fait de recirculer un fluide de recirculation chaud avec une composition équivalente à la composition au sein du réacteur 205 à travers le réacteur 205 de méthanation n'a aucune incidence sur les équilibres thermique et thermochimique de cette réaction mais permet de maintenir un débit hydrodynamique stable.

Cette recirculation est régulée par une boucle de régulation comportant le moyen de mesure 230 de débit de syngas en entrée 210 du réacteur 205 et le moyen de commande 235 du recirculateur 225.

Le moyen de mesure 230 est, par exemple, un débitmètre configuré pour réaliser une mesure du débit volumique de syngas traversant l'entrée 210 en amont de la recirculation des produits de réaction chaud.

Le moyen de commande 235 est, par exemple, un circuit électronique de commande configuré pour comparer le débit mesuré et une troisième valeur prédéterminée, correspondant au débit nominal de conception du réacteur 205 de méthanation.

En fonction du décalage détecté, le moyen de commande 235 commande la recirculation de tout ou partie du flux de produits de la réaction de méthanation dans le réacteur 205 pour que le débit en entrée 210 de ce réacteur 205 soit proche du débit nominal de conception de ce réacteur 205.

La mise en oeuvre de ces modes de réalisation permet d'assurer une hydrodynamique stable au sein du réacteur 205. Ainsi, le taux de conversion du gaz de synthèse en méthane de synthèse qui dépend fortement du ratio débit de gaz de synthèse sur masse de catalyseur est toujours maintenue constante.

Dans des modes de réalisation préférentiels, tel que celui représenté en figure 1, le dispositif 200 comporte un séparateur 245 d'eau contenue dans les produits de méthanation.

Ce séparateur 245 est configuré pour refroidir le gaz de synthèse en-dessous du point de rosée de l'eau qui est séparée en grande partie du flux de produits de réaction sous forme liquide. La chaleur habituellement extraite au sein du réacteur 205 de méthanation est également extraite et potentiellement valorisé dans ce même séparateur 245. Le refroidissement du gaz au travers du séparateur 245 est compris entre -5 et 60°C, et de préférence entre 5 et 40°C pour éliminer plus d'eau contenu dans le gaz de synthèse.

Dans des modes de réalisation préférentiels, tel que celui représenté en figure 1, le dispositif 200 comporte :
- une deuxième canalisation 250 de recirculation, du méthane de synthèse froid en sortie du séparateur 245, reliant la sortie du séparateur 245 et l'entrée 210 du réacteur 205 de méthanation, comportant un recirculateur 255, dit « quatrième recirculateur », dudit méthane de synthèse,
- un moyen 260 de mesure de la température de réaction de méthanation à l'intérieur, ou en sortie, du réacteur 205 de méthanation et
- un moyen 265 de commande du quatrième recirculateur 255 configuré pour commander la recirculation d'une quantité de méthane de synthèse déterminée en fonction de la température mesurée.

La deuxième canalisation 250 est, par exemple, une dérivation de tout ou partie du flux sorti du séparateur 245 vers l'entrée 210 du réacteur 205. Cette deuxième canalisation 250 comporte un recirculateur 255, ce recirculateur 255 étant, par exemple :
- un surpresseur mécanique comportant un variateur de vitesse,
- un ensemble comportant un surpresseur mécanique à vitesse constante et un système de régulation de débit ou
- un ventilateur.

Ainsi, en aval de la jonction entre l'entrée 210 de syngas et la deuxième canalisation 250 de recirculation, le syngas est complété par un flux correspondant à la recirculation d'une partie du méthane de synthèse froid récupéré en sortie du séparateur 245.

Le moyen de mesure 260 de la température en sortie, ou dans, le réacteur 205 est, par exemple, une sonde de température positionnée dans la sortie 215 ou dans le réacteur 205.

Le moyen de commande 265 est, par exemple, un circuit électronique de commande configuré pour comparer la température mesurée et une valeur prédéterminée, correspondant à une température nominale de fonctionnement du réacteur 205.

La mise en oeuvre de ces modes de réalisation permet de réduire le besoin en surfaces d'échanges thermiques au sein du réacteur 205. Ainsi, la mise en oeuvre d'un réacteur 205 à lit refroidi par les parois et d'un réacteur 205 à eau bouillante deviennent de simples lits fixes et un réacteur 205 à lit fluidisé avec échangeur immergé devient un simple lit fluidisé avec suppression des contraintes d'intégration des tubes notamment lorsque la pression opératoire est élevée.

La mise en oeuvre d'un réacteur sans échangeur pour un fonctionnement isotherme n'est possible que si le débit de recirculation à travers la deuxième canalisation 250 est suffisamment élevé pour permettre l'évacuation de la totalité de la chaleur de la réaction de méthanation.

La recirculation du gaz de synthèse refroidi à un double effet sur la composition du gaz de synthèse produit et sur le système de refroidissement du réacteur 205.

Dans des modes de réalisation préférentiels, tel que celui représenté en figure 1, le dispositif 200 comporte :
- un moyen 270 de mesure d'une caractéristique de composition du méthane de synthèse sorti du réacteur 205 de méthanation et
- un moyen 275 de régulation d'un débit de CO₂ injecté dans l'entrée 110 pour syngas du réacteur 205 d'hydrogénation, ce moyen de régulation étant commandé en fonction de la caractéristique mesurée et d'une quatrième valeur 280 prédéterminée.

Le moyen de mesure 270 est, par exemple, configuré pour mesurer le PCS ou la composition du méthane de synthèse. Un tel moyen de mesure 270 est, par exemple :
- un chromatographe configuré pour réaliser une chromatographie en phase gazeuse pour mesurer sa composition (CH₄, CₙHₘ, H₂, CO, CO₂, N₂) et un circuit électronique pour calculer le PCS du méthane de synthèse en fonction de cette composition ou
- un « calorimètre » pour mesurer directement le PCS du méthane de synthèse.

Le moyen de régulation 275 est, par exemple, un circuit électronique de commande configuré pour comparer la valeur de la caractéristique de composition et la quatrième valeur 280 prédéterminée. En fonction du résultat de cette comparaison, le moyen de régulation commande l'ouverture ou la fermeture partielle ou totale d'une vanne positionnée sur l'entrée 112 pour dioxyde de carbone.

Cette chaine présente ainsi l'avantage de produire un gaz de synthèse directement aux spécifications de valorisation du gaz naturel du fait d'une régulation du débit de CO₂ en entrée.

La quatrième valeur 280 prédéterminée est fixée de telle sorte à produire un méthane de synthèse dont les caractéristiques sont proches des propriétés du gaz naturel. Ainsi, si le PCS du flux est par exemple trop faible par rapport à la quatrième valeur prédéterminée, une augmentation du débit de la vanne de régulation du flux de CO₂ est mise en place afin de permettre une conversion du H₂ en excès qui peut avoir un impact sur la qualité du méthane de synthèse. Cette régulation permet un ajustement continu de la qualité du méthane de synthèse et évite l'installation des étapes complexes et coûteuses de mises aux spécifications (membrane, absorption, etc.) pour séparer le H₂ et/ou CO₂ en excès. L'action sur le flux de CO₂ plutôt que sur H₂ est préférée car la production d'hydrogène H₂ est généralement fonction de la quantité d'électricité à bas coût disponible sur le réseau alors que CO₂ est un réactif connexe disponible dans des proportions généralement plus importantes.

Dans des modes de réalisation préférentiels, tel que celui représenté en figure 1, le dispositif 200 comporte un détendeur 285 du syngas entre le dispositif 100 d'hydrogénation du CO₂ pour produire du méthanol et l'entrée 210 du réacteur 205 de méthanation ou en aval du réacteur 205 de méthanation.

La pression d'utilisation (injection, mobilité) du méthane de synthèse n'est pas toujours en accord avec la pression de fonctionnement du dispositif 100. Ainsi, suivant la valorisation escomptée pour le méthane de synthèse, une étape d'ajustement de la pression peut être nécessaire. En figure 21, le détendeur 285 est positionné en amont du réacteur 205 de méthanation. Il est cependant possible de réaliser cette détente après la réaction de méthanation et dans ce cas, la réaction de méthanation doit être assurée à une pression très élevée (supérieure à 70 bars) avec les difficultés de conception mécanique que cela peut engendrer. Si le choix se porte sur la production d'un méthane de synthèse à une pression opératoire plus faible que celle de l'étape d'hydrogénation, une détente du syngas en excès est requise. Ce laminage peut conduire à un refroidissement brusque du gaz de synthèse par effet Joule-Thompson. Cet effet est notamment plus ou moins marqué en fonction du taux de détente appliqué. Parfois, des températures négatives peuvent être atteintes et conduire à la perte d'une partie non négligeable de la contenance en CO₂ du flux de syngas avec accumulation de liquide ou de givre. Une solution peut consister à subir ces changements de phase et l'autre à assurer un préchauffage amont suffisant pour l'éviter.

Dans des modes de réalisation préférentiels, tel que celui représenté en figure 1, le dispositif 200 comporte, en amont du détendeur 285, un échangeur 290 de chaleur dont la température de sortie est déterminée en fonction d'une température du syngas mesurée, par un moyen 295 de mesure de température, en aval du détendeur.

Cet échangeur 290 et le système de contrôle de la température qui y est associé ne sont pas requis lorsque la détente 285 est réalisée en aval de la méthanation 205.

Dans ces modes de réalisation, le flux est tout d'abord préchauffé au travers d'un échangeur 290 puis détendu à une pression légèrement supérieure à la pression d'utilisation visée du méthane de synthèse (injection, mobilité) afin de compenser les pertes de charges au travers des différents équipements jusqu'au poste de valorisation du méthane de synthèse. Le préchauffage est configuré pour permettre d'atteindre, en sortie du détendeur 285, une température supérieure à la température de rosée du CO₂ dans les conditions de pression considérées.

La valeur de la température de sortie de l'échangeur 290 est déterminée en fonction de la température mesurée par le moyen de mesure 295 de température. Ce moyen de mesure 295 est, par exemple, une sonde de température positionnée dans la conduite de sortie du détendeur 285.

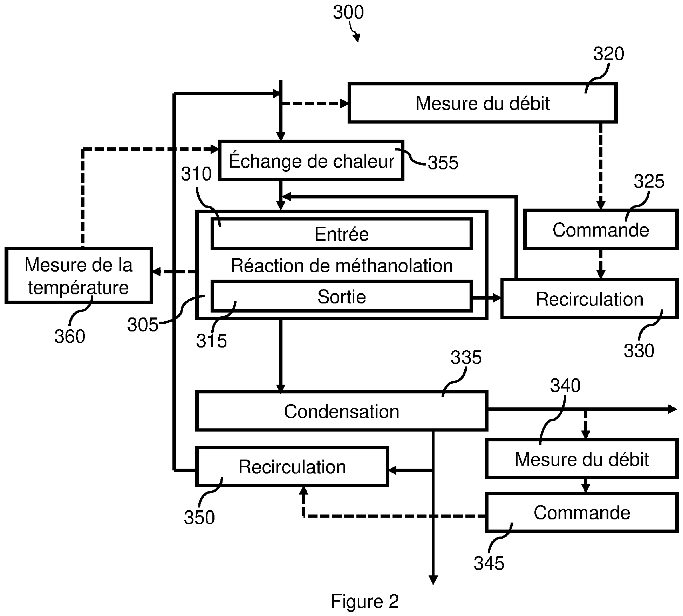

On observe, en figure 2, schématiquement et sous forme d'un logigramme d'étapes, un mode de réalisation particulier du procédé 300 objet de la présente invention. Ce procédé 300 d'hydrogénation du CO₂ pour produire du méthanol à partir d'un syngas comportant :
- du dihydrogène, H₂, sorti par exemple d'une étape d'électrolyse de l'eau, H₂O, et
- du dioxyde de carbone, CO₂,
comporte :
- une étape 305 de réaction d'hydrogénation du CO₂ pour produire du méthanol, dite
   « hydrogénation », dans un réacteur d'hydrogénation, comportant :
   - une étape 310 d'entrée pour le syngas et
   - une étape 315 de sortie pour des produits de la réaction d'hydrogénation comportant au moins du méthanol, CH₃OH ou MeOH,
- une étape 320 de mesure du débit de syngas traversant l'entrée du réacteur en amont d'une jonction entre une conduite de recirculation, reliant la sortie du réacteur à l'entrée du réacteur, dans une conduite de canalisation comportant un recirculateur, dit « premier recirculateur », de ladite partie des produits,
- une étape 325 de commande du premier recirculateur configuré pour commander la recirculation d'une quantité de produits chauds de la réaction d'hydrogénation déterminée en fonction du débit de syngas mesuré et d'une première valeur consigne prédéterminée et
- une première étape 330 de recirculation, d'au moins une partie des produits chauds de la réaction d'hydrogénation dans la conduite de recirculation.

Dans des modes de réalisation préférentiels, tel que celui représenté en figure 2, le procédé 300 comporte une étape 335 de condensation des produits d'hydrogénation pour condenser du méthanol et de l'eau.

Dans des modes de réalisation préférentiels, tel que celui représenté en figure 2, le procédé 300 comporte :
- une étape 340 de mesure du débit de méthanol condensé au cours de l'étape 335 de condensation,
- une étape 345 de commande d'un deuxième recirculateur, associé à une première canalisation de recirculation, pour commander la recirculation d'une quantité de produits froids en excès, non condensés, de réaction d'hydrogénation déterminée en fonction du débit de méthanol condensé mesuré et d'une deuxième valeur consigne prédéterminée et
- une étape 350 de recirculation d'une partie des produits froids d'hydrogénation dans la première canalisation, reliant la sortie de l'étape de condensation et l'entrée 310 de l'étape 305 de réaction d'hydrogénation, vers un point situé en amont du point de mesure du débit de syngas.

Dans des modes de réalisation préférentiels, tel que celui représenté en figure 2, le procédé 300 comporte :
- une étape de capture 360 de température de réaction dans la, ou en sortie de la, réaction 305 d'hydrogénation et
- une étape 355 d'échange de chaleur, en entrée 310 de la réaction 305, dont la température de sortie est déterminée en fonction de la température de réaction captée.

Ce procédé 300 est réalisé, par exemple, par la mise en oeuvre du dispositif 100 tel que décrit en regard de la figure 1.

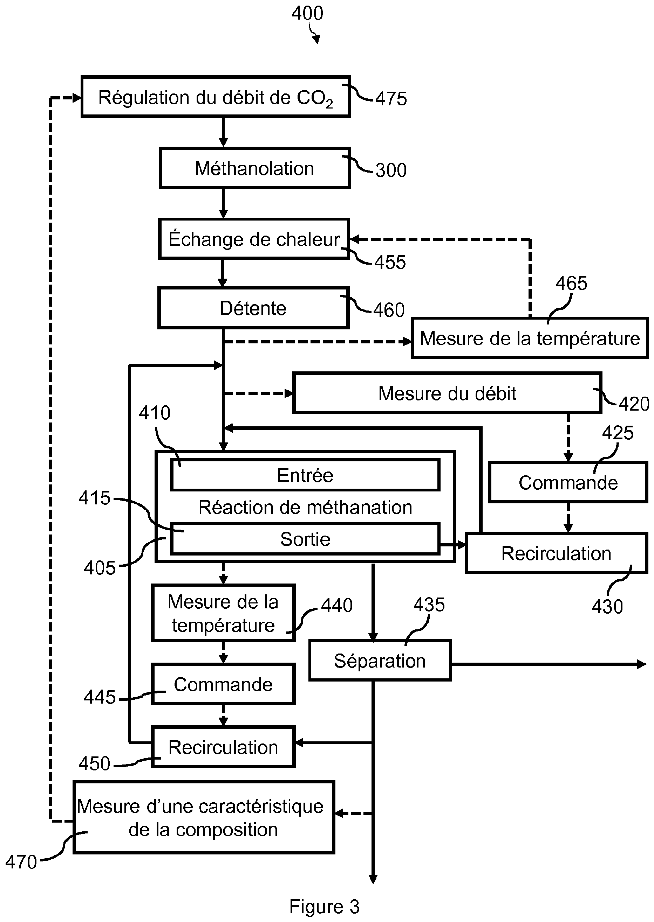

On observe, en figure 3, schématiquement et sous forme d'un logigramme d'étapes, un mode de réalisation particulier du procédé 400 objet de la présente invention. Ce procédé 400 de cogénération de méthanol et de méthane de synthèse, comporte :
- un procédé 300 d'hydrogénation du CO₂ pour produire du méthanol tel que décrit en regard de la figure 2,
- une étape 405 de réaction de méthanation, réalisée par un réacteur de méthanation, comportant :
   - une étape 410 d'entrée pour une partie des produits d'hydrogénation comportant au moins du syngas en excès suite à la réaction d'hydrogénation et
   - une étape 415 de sortie pour des produits de réaction de méthanation comportant au moins du méthane de synthèse.

Dans des modes de réalisation préférentiels, tel que celui représenté en figure 3, le procédé 400 comporte :
- une étape 420 de mesure du débit de syngas traversant l'entrée du réacteur de méthanation positionné en amont d'une jonction entre une deuxième conduite de recirculation reliant la sortie du réacteur de méthanation à l'entrée du réacteur de méthanation, comportant un recirculateur, dit « troisième recirculateur »,
- une étape 425 de commande du troisième recirculateur configuré pour commander la recirculation d'une quantité de produits de réaction de méthanation déterminée en fonction du débit de syngas mesuré et d'une troisième valeur consigne prédéterminée et
- une deuxième étape 430 de recirculation, d'au moins une partie des produits chauds de la réaction de méthanation dans la deuxième conduite de recirculation.

Dans des modes de réalisation préférentiels, tel que celui représenté en figure 3, le procédé 400 comporte une étape 435 de séparation d'eau contenue dans les produits de méthanation.

Dans des modes de réalisation préférentiels, tel que celui représenté en figure 3, le procédé 400 comporte :
- une étape 440 de mesure de la température de réaction de méthanation à l'intérieur, ou en sortie, du réacteur 405 de méthanation,
- une étape 445 de commande d'un quatrième recirculateur, associé à une deuxième canalisation de recirculation, pour commander la recirculation d'une quantité de méthane de synthèse déterminée en fonction de la température mesurée et
- une étape 450 de recirculation du gaz de synthèse froid dans la deuxième canalisation de recirculation, reliant la sortie de l'étape de séparation 435 et l'entrée 410 de la réaction 405 de méthanation.

Dans des modes de réalisation préférentiels, tel que celui représenté en figure 3, le procédé 400 comporte :
- une étape 470 de mesure d'une caractéristique de composition du méthane de synthèse sorti de la réaction 405 de méthanation et
- une étape 475 de régulation d'un débit de CO₂ injecté dans l'entrée 310 pour syngas du réacteur 305 d'hydrogénation, cette étape de régulation étant commandé en fonction de la caractéristique mesurée et d'une quatrième valeur prédéterminée.

Dans des modes de réalisation préférentiels, tel que celui représenté en figure 3, le procédé 400 comporte une étape 460 de détente du syngas entre le procédé 200 d'hydrogénation et l'entrée 410 de la réaction 405 de méthanation ou en aval de la réaction 405 de méthanation.

Dans des modes de réalisation préférentiels, tel que celui représenté en figure 3, le procédé 400 comporte, en amont de l'étape de détente 460, une étape d'échange 455 de chaleur dont la température de sortie est déterminée en fonction d'une température du syngas mesurée, au cours d'une étape 465 de mesure de température, en aval de l'étape de détente 460. Ce procédé 400 est réalisé, par exemple, par la mise en oeuvre du dispositif 200 tel que décrit en regard de la figure 1.

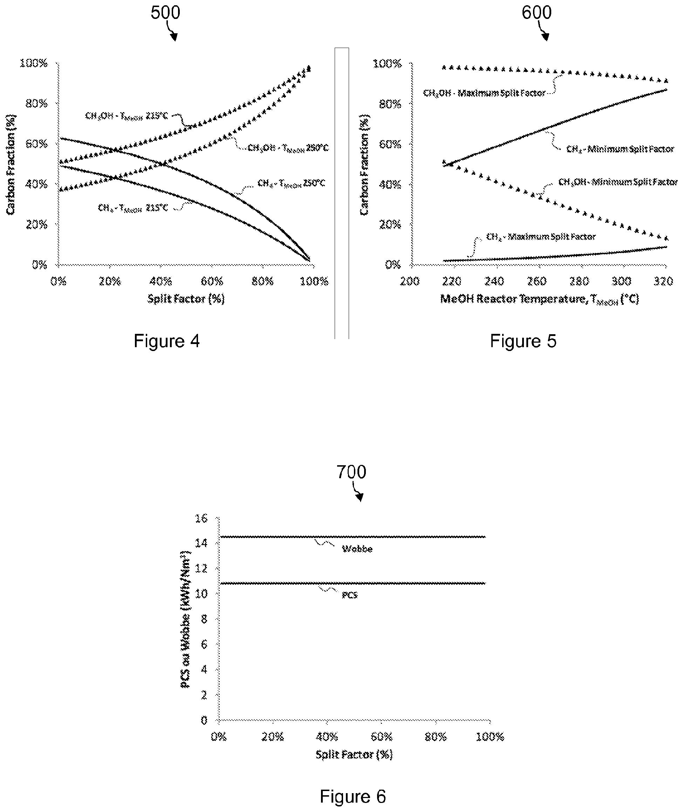

Les figures 4 à 10 visent à permettre l'évaluation de faisabilité du procédé 400 objet de la présente invention. Les figures 4 et 5 présentent l'évolution de la fraction molaire de carbone convertie en méthane et en méthanol en fonction du ratio de recirculation aussi appelé « split factor ». Ce ratio correspond à la fraction molaire de syngas froid en excès après l'étape de condensation 145, recirculé par la première canalisation vers l'entrée de l'échangeur situé en amont du réacteur d'hydrogénation 105. Pour illustrer, un split factor de 20% signifie que 80 % du flux molaire de syngas en excès à la réaction d'hydrogénation est dédié à la production de méthane de synthèse alors que 20 % de ce même flux est recirculé en amont du réacteur d'hydrogénation pour répondre à la demande en méthanol. L'ajustement de ce « split factor » est un élément clé pour adapter la production du procédé objet de la présente invention à la demande en méthanol du marché.

L'exemple illustré en figures 4 et 5 montre que la mise oeuvre du procédé et du dispositif objets de la présente invention permettent de flexibiliser la production de méthanol en agissant sur le ratio de recirculation du syngas en excès. Ainsi, pour un réacteur d'hydrogénation opérant à 80 bars et 250°C, la rangeabilité du système sur le flux de méthanol se situe entre 37 et 97% de conversion du carbone entrant sous forme de CO₂ par le flux 112 en méthanol. Pour élargir ou réduire ce domaine, il est par ailleurs possible d'agir sur la température et/ou la pression opératoire du réacteur d'hydrogénation, comme illustré en figure 5. Ainsi, une augmentation de la température jusqu'à 320°C permet d'élargir la rangeabilité entre 13 et 91 % de conversion du carbone entrant sous forme de CO₂ par le flux 112 en méthanol. La baisse de production de méthanol s'explique principalement par l'effet défavorable de la température sur l'équilibre thermochimique d'hydrogénation. Le syngas en excès est transformé en méthane de synthèse dans le bloc aval de méthanation. La qualité de ce méthane de synthèse et sa conformité vis-à-vis des spécifications (PCS, Wobbe, H₂, CO₂, etc.) sont assurées par la régulation du débit de CO₂ sur le PCS ou la composition. Par ce biais, le PCS et l'indice de Wobbe, illustrés en figure 6, sont maintenus constants pour toute variation du ratio de recirculation et/ou des conditions opératoires (pressions, températures). Le maintien de l'hydrodynamique des réacteurs est obtenu grâce aux recirculations de produits chauds pour l'hydrogénation et pour la méthanation.

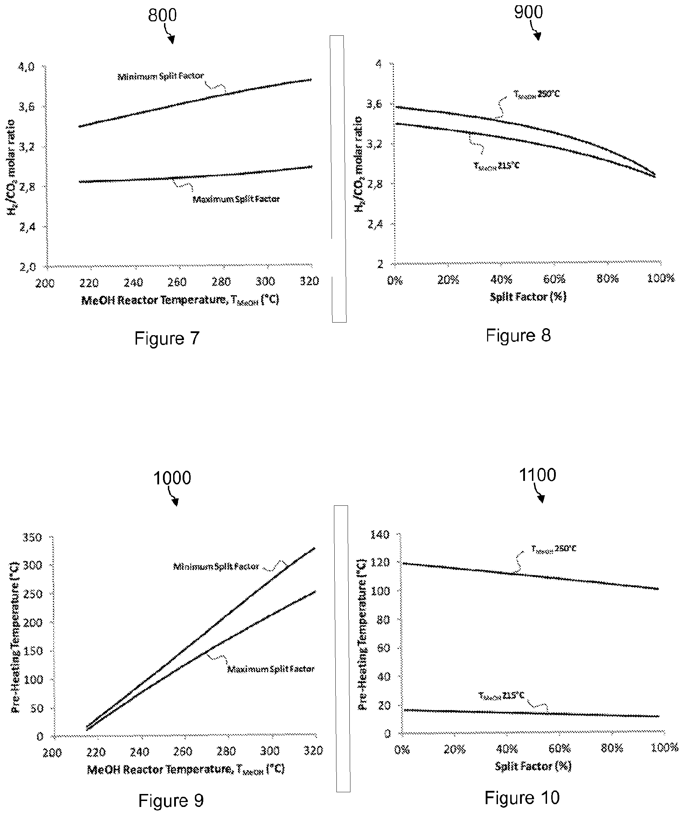

La régulation du débit de CO₂ revient à ajuster le ratio H₂/CO₂ alimentant le procédé. Les figures 7 et 8 représentent l'évolution de ce ratio pour différents taux de production de méthanol. Il varie entre 2,8 et 3 lorsque le « split factor » est proche de l'unité et des températures comprises entre 215°C et 320°C, à une pression égale à 80 bars. Quand quasi-aucune recirculation n'est assurée, ce ratio doit être ajusté entre 3,3 et 3,9 à une pression de 80 bars.

La principale fonction de l'échangeur de chaleur positionné en amont du réacteur d'hydrogénation est de maintenir l'isothermicité du réacteur sans immersion de tubes d'échange dans la couche catalytique. En fonction de la demande en méthanol (action sur le ratio de recirculation), des conditions opératoires (températures et pressions), et de la température d'alimentation du mélange H₂ - CO₂, cet échange doit permettre de réguler la température du flux entrant dans le réacteur d'hydrogénation. Les figures 9 et 10 visent à visualiser le domaine de température à assurer en sortie de cet échangeur pour garantir l'isothermicité du réacteur d'hydrogénation. Lorsque le réacteur fonctionne à basse température, les conversions élevées de carbone en méthanol dans la première étape induisent un fort dégagement de chaleur nécessitant un refroidissement intense. Au contraire, l'élévation de la température réactionnelle réduit considérablement ces conversions et le système peut devenir déficient en énergie impliquant un besoin de préchauffage amont pour maintenir l'isothermicité. Ce manque est illustré en figure 9 à haute température de réaction (320°C) et faible facteur de split qui correspond à la plus faible production en méthanol. Pour ce cas de figure, la température de sortie de l'étape d'échange de chaleur est de 325°C soit supérieure à la température opératoire du réacteur.

Ainsi, comme on le comprend à la lecture de la présente description, le procédé autant que le dispositif objet de la présente invention présente une très grande flexibilité. Ce procédé et ce dispositif permettent notamment d'ajuster de manière simple la production de méthanol à la demande tout en lissant la disponibilité de l'électricité renouvelable. Par ailleurs, les réacteurs mis en oeuvre pour l'application de ce procédé ne requièrent pas l'intégration complexe d'échangeurs dans la couche catalytique. L'objet de la présente invention peut donc s'intégrer parfaitement à une filière « intermittente » tel que le power to gas/liquid.

## Revendications

1. Dispositif (200) de cogénération de méthanol et de méthane de synthèse, **caractérisé en ce qu'**il comporte :
- un dispositif (100) d'hydrogénation du CO₂ pour produire du méthanol à partir d'un syngas comportant :
- du dihydrogène, H₂, sorti par exemple d'un dispositif d'électrolyse de l'eau, H₂O, et
- du dioxyde de carbone, CO₂,
ledit dispositif d'hydrogénation comportant :
- un réacteur (105) d'hydrogénation du CO₂ pour produire du méthanol, dite
« hydrogénation », comportant :
- une entrée (110) pour le syngas et
- une sortie (115) pour des produits de la réaction d'hydrogénation comportant au moins du méthanol, CH₃OH ou MeOH,
- une première conduite (120) de recirculation, d'une partie des produits chauds de la réaction d'hydrogénation reliant la sortie du réacteur à l'entrée du réacteur, comportant un recirculateur (125), dit « premier recirculateur », de ladite partie des produits de réaction,
- un moyen (130) de mesure du débit de syngas traversant l'entrée du réacteur positionné en amont d'une jonction entre la conduite de recirculation et l'entrée du réacteur et
- un moyen (135) de commande du premier recirculateur configuré pour comparer le débit mesuré et une première valeur prédéterminée, correspondant au débit nominal de conception du réacteur (105) d'hydrogénation, en fonction du décalage détecté, et commander la recirculation de tout ou partie du flux de produits chauds de la réaction d'hydrogénation dans le réacteur (105) pour que le débit en entrée (110) de ce réacteur (105) soit proche du débit nominal de conception de ce réacteur (105), et
le dispositif d'hydrogénation comportant, de plus :
- un condenseur (145), des produits de réaction d'hydrogénation sortis du réacteur (105), positionné en aval d'une jonction entre la conduite (120) de recirculation et la sortie (115) du réacteur (105) d'hydrogénation, pour séparer au moins le méthanol et l'eau du syngas en excès suite à la réaction d'hydrogénation,
- une première canalisation (150) de recirculation, d'une partie du syngas froid en excès en sortie du condenseur (145), reliant la sortie (155) du condenseur et l'entrée (110) du réacteur (105) d'hydrogénation, en amont du point de mesure du débit de syngas par le moyen (130) de mesure, comportant un recirculateur (160), dit « deuxième recirculateur », dudit syngas,
- un moyen (165) de mesure du débit du méthanol condensé par le condenseur (145),
- un moyen (170) de commande du deuxième recirculateur configuré pour comparer le débit mesuré et une deuxième valeur prédéterminée, correspondant à une consommation de méthanol mesurée en aval du dispositif (100) ou à une consigne de production de méthanol, et
- un réacteur (205) de méthanation en aval du dispositif d'hydrogénation comportant :
- une entrée (210) pour une partie du syngas en excès suite à la réaction d'hydrogénation et en aval d'une jonction entre la sortie du condenseur et la première conduite de recirculation, d'une partie du syngas froid en excès en sortie du condenseur (145) et
- une sortie (215) pour des produits de réaction de méthanation comportant au moins du méthane de synthèse.

2. Dispositif (100) selon la revendication 1, dans lequel le dispositif d'hydrogénation comporte un capteur (180) de température de réaction dans le, ou en sortie du, réacteur (105) d'hydrogénation, l'entrée (110) pour syngas du réacteur comportant un échangeur (185) de chaleur dont la température de sortie est déterminée en fonction de la température de réaction captée.

3. Dispositif (200) selon l'une des revendications 1 ou 2, qui comporte :
- une deuxième conduite (220) de recirculation, d'une partie des produits chauds de la réaction de méthanation reliant la sortie du réacteur de méthanation à l'entrée du réacteur de méthanation, comportant un recirculateur (225), dit « troisième recirculateur », de ladite partie des produits,
- un moyen (230) de mesure du débit de syngas traversant l'entrée (210) du réacteur (205) de méthanation positionné en amont d'une jonction entre la deuxième conduite de recirculation et l'entrée du réacteur de méthanation et
- un moyen (235) de commande du troisième recirculateur configuré pour commander la recirculation d'une quantité partielle de produits de réaction de méthanation déterminée en fonction du débit de syngas mesuré et d'une troisième valeur (240) consigne prédéterminée.

4. Dispositif (200) selon l'une des revendications 1 à 3, qui comporte un séparateur (245) d'eau contenue dans les produits de méthanation.

5. Dispositif (200) selon la revendication 4, qui comporte :
- une deuxième canalisation (250) de recirculation, du méthane de synthèse froid en sortie du séparateur, reliant la sortie du séparateur et l'entrée du réacteur de méthanation, comportant un recirculateur (255), dit « quatrième recirculateur », dudit méthane de synthèse,
- un moyen (260) de mesure de la température de réaction de méthanation à l'intérieur, ou en sortie, du réacteur (205) de méthanation et
- un moyen (265) de commande du quatrième recirculateur configuré pour commander la recirculation d'une quantité de méthane de synthèse déterminée en fonction de la température (260) mesurée.

6. Dispositif (200) selon l'une des revendications 1 à 5, qui comporte :
- un moyen (270) de mesure d'une caractéristique de composition du méthane de synthèse sorti du réacteur (205) de méthanation et
- un moyen (275) de régulation d'un débit de CO₂ injecté dans l'entrée (110) pour syngas du réacteur (205) d'hydrogénation, ce moyen de régulation étant commandé en fonction de la caractéristique mesurée et d'une quatrième valeur (280) prédéterminée.

7. Dispositif (200) selon l'une des revendications 1 à 6, qui comporte un détendeur (285) du syngas entre le dispositif (100) d'hydrogénation et l'entrée (210) du réacteur (205) de méthanation ou en aval du réacteur (205) de méthanation.

8. Dispositif (200) selon la revendication 7, qui comporte, en amont du détendeur (285), un échangeur (290) de chaleur dont la température de sortie est déterminée en fonction d'une température du syngas mesurée, par un moyen (295) de mesure de température, en aval du détendeur.

9. Procédé (400) de cogénération de méthanol et de méthane de synthèse mis en oeuvre par un dispositif selon l'une des revendications 1 à 8, **caractérisé en ce qu'**il comporte :
- un procédé (300) d'hydrogénation du CO₂ pour produire du méthanol pour produire du méthanol à partir d'un syngas comportant :
- du dihydrogène, H₂, sorti par exemple d'une étape d'électrolyse de l'eau, H₂O, et
- du dioxyde de carbone, CO₂,
ledit procédé d'hydrogénation comportant :
- une étape (305) de réaction d'hydrogénation du CO₂ pour produire du méthanol, dite « hydrogénation », dans un réacteur d'hydrogénation, comportant :
- une étape (310) d'entrée pour le syngas et
- une étape (315) de sortie pour des produits de la réaction d'hydrogénation comportant au moins du méthanol, CH₃OH ou MeOH,
- une étape (320) de mesure du débit de syngas traversant l'entrée du réacteur en amont d'une jonction entre une conduite de recirculation, reliant la sortie du réacteur à l'entrée du réacteur, dans une conduite de canalisation comportant un recirculateur, dit « premier recirculateur », d'une partie des produits chauds de la réaction d'hydrogénation proche d'un débit nominal de conception du réacteur d'hydrogénation,
- une étape (325) de commande du premier recirculateur configuré pour commander la recirculation d'une quantité partielle de produits de réaction d'hydrogénation déterminée, dans laquelle le débit mesuré et une première valeur prédéterminée, correspondant au débit nominal de conception du réacteur (105) d'hydrogénation, sont mesurées et en fonction du décalage détecté, et dans laquelle la recirculation de tout ou partie du flux de produits chauds de la réaction d'hydrogénation dans le réacteur (105) pour que le débit en entrée (110) de ce réacteur (105) soit proche du débit nominal de conception de ce réacteur (105) est commandée,
le procédé d'hydrogénation comportant, de plus :
- une étape (335) de condensation des produits d'hydrogénation pour condenser et séparer au moins le méthanol et l'eau du syngas en excès suite à la réaction d'hydrogénation,
- une étape (340) de mesure du débit de méthanol condensé au cours de l'étape (335) de condensation,
- une étape (345) de commande d'un deuxième recirculateur, associé à une première canalisation de recirculation, pour commander la recirculation d'une quantité partielle de produits froids en excès, non condensés, de réaction d'hydrogénation, dans laquelle le débit mesuré et une deuxième valeur prédéterminée sont comparés, la deuxième valeur limite prédéterminée correspondant à une consommation de méthanol mesurée en aval du dispositif (100) ou à une consigne de production de méthanol,
- une étape (350) de recirculation d'une partie du syngas froid en excès en sortie de l'étape de condensation, dans la première canalisation, reliant la sortie de l'étape de condensation et l'entrée (310) de l'étape (305) de réaction d'hydrogénation, vers un point situé en amont du point de mesure du débit de syngas et
- une étape (405) de réaction de méthanation, réalisée par un réacteur de méthanation, comportant :
- une étape (410) d'entrée pour une partie du syngas en excès suite à la réaction d'hydrogénation et après l'étape (350) de recirculation d'une partie des produits froids d'hydrogénation dans la première canalisation et
- une étape (415) de sortie pour des produits de réaction de méthanation comportant au moins du méthane de synthèse.

10. Procédé (400) selon la revendication 9, qui comporte :
- une étape (420) de mesure du débit de syngas traversant l'entrée du réacteur de méthanation positionné en amont d'une jonction entre une deuxième conduite de recirculation reliant la sortie du réacteur de méthanation à l'entrée du réacteur de méthanation, comportant un recirculateur, dit « troisième recirculateur »,
- une étape (425) de commande du troisième recirculateur configuré pour commander la recirculation d'une quantité partielle de produits de réaction de méthanation déterminée en fonction du débit de syngas mesuré et d'une troisième valeur consigne prédéterminée et
- une deuxième étape (430) de recirculation, d'une partie des produits chauds de la réaction de méthanation dans la deuxième conduite de recirculation.

## Patentansprüche

1. Vorrichtung (200) für die gleichzeitige Erzeugung von Methanol und synthetischem Methan, **dadurch gekennzeichnet, dass** sie umfasst:
- eine Vorrichtung (100) zur Hydrierung von CO₂ zur Erzeugung von Methanol aus einem Syngas mit:
- Diwasserstoff, H₂, z. B. aus einer Wasserelektrolysevorrichtung, H₂O, und
- Kohlendioxid, CO₂,
wobei die Hydriervorrichtung aufweist:
- einen CO₂-Hydrierungsreaktor (105) zur Erzeugung von Methanol, genannt "Hydrierung", umfassend:
- einen Eingang (110) für das Syngas und
- einen Ausgang (115) für Hydrierungsreaktionsprodukte, die mindestens Methanol, CH₃OH oder MeOH enthalten,
- eine erste Umlaufleitung (120) eines Teils der heißen Produkte der Hydrierungsreaktion, die den Reaktorausgang mit dem Reaktoreingang verbindet, die einen Umlaufkörper (125), den sogenannten "ersten Umlaufkörper", des besagten Teils der Reaktionsprodukte umfasst,
- ein Mittel (130) zur Messung des Syngasdurchsatzes durch den Reaktoreingang, das einer Verbindung zwischen der Umlaufleitung und dem Reaktoreingang vorgeschaltet ist, und
- ein Mittel (135) zur Steuerung des ersten Umlaufkörpers, das so eingerichtet ist, dass es den gemessenen Durchsatz und einen ersten vorbestimmten Wert vergleicht, der dem nominellen Auslegungsdurchsatz des Hydrierungsreaktors (105) entspricht, in Abhängigkeit von der erkannten Abweichung, und den Umlauf des gesamten oder eines Teils des Stroms von heißen Produkten der Hydrierungsreaktion im Reaktor (105) steuert, sodass der Eingangsdurchsatz (110) dieses Reaktors (105) etwa dem Auslegungsdurchsatz dieses Reaktors (105) entspricht, und
wobei die Hydriervorrichtung zusätzlich aufweist:
- einen Kondensator (145) der Hydrierungsreaktionsprodukte aus dem Reaktor (105), der stromabwärts einer Verbindung zwischen der Umlaufleitung (120) und dem Ausgang (115) des Hydrierungsreaktors (105) positioniert ist, um zumindest das Methanol und das Wasser aus dem überschüssigen Syngas infolge der Hydrierungsreaktion zu trennen,
- eine erste Umlaufleitung (150) für einen Teil des überschüssigen kalten Syngases am Ausgang des Kondensators (145), die den Ausgang (155) des Kondensators und den Eingang (110) des Hydrierungsreaktors (105) verbindet, vor dem Messpunkt des Syngasdurchsatzes durch das Messmittel (130), das einen Umlaufkörper (160), den sogenannten "zweiten Umlaufkörper", des Syngases umfasst,
- ein Mittel (165) zur Messung des Durchsatzes des durch den Kondensator (145) kondensierten Methanols,
- ein Mittel (170) zur Steuerung des zweiten Umlaufkörpers, das so eingerichtet ist, dass es den gemessenen Durchsatz mit einem zweiten vorbestimmten Wert vergleicht, der einem nach der Vorrichtung (100) gemessenen Methanolverbrauch oder einem Methanol-Produktionssollwert entspricht, und
- einen Methanisierungsreaktor (205) stromabwärts der Hydriervorrichtung, der aufweist:
- einen Eingang (210) für einen Teil des überschüssigen Syngases infolge der Hydrierungsreaktion und nach einer Verbindung zwischen dem Kondensatorausgang und der ersten Umlaufleitung, eines Teils des überschüssigen kalten Syngases am Kondensatorausgang (145) und
- einen Ausgang (215) für Methanisierungsreaktionsprodukte, die mindestens synthetisches Methan enthalten.

2. Vorrichtung (100) nach Anspruch 1, wobei die Hydriervorrichtung einen Sensor (180) für die Reaktionstemperatur im oder am Ausgang des Hydrierreaktors (105) umfasst, wobei der Eingang (110) für Syngas des Reaktors einen Wärmetauscher (185) umfasst, dessen Ausgangstemperatur in Abhängigkeit von der erfassten Reaktionstemperatur bestimmt wird.

3. Vorrichtung (200) nach einem der Ansprüche 1 oder 2, die aufweist:
- eine zweite Umlaufleitung (220) eines Teils der heißen Produkte der Methanisierungsreaktion, die den Ausgang des Methanisierungsreaktors mit dem Eingang des Methanisierungsreaktors verbindet, die einen Umlaufkörper (225), den sogenannten "dritten Umlaufkörper", des besagten Teils der Produkte umfasst,
- ein Mittel (230) zur Messung des Syngasdurchsatzes durch den Eingang (210) des Methanisierungsreaktors (205), das vor einer Verbindung zwischen der zweiten Umlaufleitung und dem Eingang des Methanisierungsreaktors positioniert ist, und
- ein Mittel (235) zur Steuerung des dritten Umlaufkörpers, das so eingerichtet ist, dass es den Umlauf einer Teilmenge von Methanierungsreaktionsprodukten steuert, die in Abhängigkeit vom gemessenen Synthesegasdurchsatz und einem dritten vorbestimmten Sollwert (240) bestimmt wird.

4. Vorrichtung (200) nach einem der Ansprüche 1 bis 3, die einen Abscheider (245) für in den Methanierungsprodukten enthaltenes Wasser aufweist.

5. Vorrichtung (200) nach Anspruch 4, die umfasst:
- eine zweite Umlaufleitung (250) von kaltem synthetischem Methan am Ausgang des Abscheiders, die den Ausgang des Abscheiders und den Eingang des Methanisierungsreaktors verbindet, die einen Umlaufkörper (255), den sogenannten "vierten Umlaufkörper", des synthetischen Methans aufweist,
- ein Mittel (260) zur Messung der Methanisierungsreaktionstemperatur im Inneren oder am Ausgang des Methanisierungsreaktors (205) und
- ein Mittel (265) zur Steuerung des vierten Umlaufkörpers, das so eingerichtet ist, dass es den Umlauf einer bestimmten Menge synthetischen Methans in Abhängigkeit von der gemessenen Temperatur (260) steuert.

6. Vorrichtung (200) nach einem der Ansprüche 1 bis 5, die aufweist:
- ein Mittel (270) zur Messung eines Zusammensetzungsmerkmals des synthetischen Methans aus dem Methanisierungsreaktor (205) und
- ein Mittel (275) zur Regelung eines in den Eingang (110) für Syngas des Hydrierungsreaktors (205) eingespeisten CO₂-Durchsatzes, wobei dieses Regelmittel in Abhängigkeit von dem gemessenen Merkmal und einem vierten vorbestimmten Wert (280) gesteuert wird.

7. Vorrichtung (200) nach einem der Ansprüche 1 bis 6, die einen Druckminderer (285) des Syngases zwischen der Hydriervorrichtung (100) und dem Eingang (210) des Methanisierungsreaktors (205) oder nach dem Methanisierungsreaktor (205) umfasst.

8. Vorrichtung (200) nach Anspruch 7, die vor dem Druckminderer (285) einen Wärmetauscher (290) aufweist, dessen Ausgangstemperatur in Abhängigkeit von einer gemessenen Syngastemperatur durch ein nach dem Druckminderer stromabwärts befindliches Mittel (295) zur Temperaturmessung bestimmt wird.

9. Verfahren (400) für die gleichzeitige Erzeugung von Methanol und synthetischem Methan, das in einer Vorrichtung nach einem der Ansprüche 1 bis 8 durchgeführt wird, **dadurch gekennzeichnet, dass** es aufweist:
- ein Verfahren (300) zur Hydrierung von CO₂ zur Erzeugung von Methanol zur Erzeugung von Methanol aus einem Syngas mit:
- Diwasserstoff, H₂, z. B. aus einer Wasserelektrolyse, H₂O, und
- Kohlendioxid, CO₂,
wobei das Hydrierungsverfahren aufweist:
- einen Hydrierungsreaktionsschritt (305) von CO₂ zur Erzeugung von Methanol, genannt "Hydrierung", in einem Hydrierungsreaktor, umfassend:
- einen Eingangsschritt (310) für das Syngas und
- einen Ausgangsschritt (315) für Hydrierungsreaktionsprodukte, die mindestens Methanol, CH₃OH oder MeOH enthalten,
- einen Messschritt (320) des Syngasdurchsatzes, der durch den Reaktoreinlass vor einer Verbindung zwischen einer Rezirkulationsleitung fließt, die den Reaktorausgang mit dem Reaktoreinlass verbindet, in einer Umlaufleitung, die einen Umlaufkörper, den sogenannten "ersten Umlaufkörper", eines Teils der heißen Produkte der Hydrierungsreaktion nahe einem nominellen Auslegungsdurchsatz des Hydrierungsreaktors umfasst,
- einen Steuerschritt (325) des ersten Umlaufkörpers, der so eingerichtet ist, dass er den Umlauf einer bestimmten Teilmenge von Hydrierungsreaktionsprodukten steuert, bei dem der gemessene Durchsatz und ein erster vorbestimmter Wert, der dem nominellen Auslegungsdurchsatz des Hydrierungsreaktors (105) entspricht, gemessen werden und in Abhängigkeit von der erkannten Abweichung, und den Umlauf des gesamten oder eines Teils des Stroms von heißen Produkten der Hydrierungsreaktion im Reaktor (105), sodass der Eingangsdurchsatz (110) dieses Reaktors (105) etwa dem Auslegungsdurchsatz dieses Reaktors (105) emtspricht, und
wobei das Hydrierungsverfahren weiterhin aufweist:
- einen Kondensationsschritt (335) der Hydrierungsprodukte, um wenigstens das Methanol und das Wasser aus dem überschüssigen Syngas infolge der Hydrierungsreaktion zu kondensieren und zu trennen,
- einen Messschritt (340) der Menge des während des Kondensationsschritts (335) kondensierten Methanols,
- einen Steuerschritt (345) eines zweiten Umlaufkörpers, der mit einer ersten Umlaufleitung verbunden ist, um den Umlauf einer Teilmenge überschüssiger, nicht kondensierter kalter Hydrierungsreaktionsprodukte zu steuern, bei dem der gemessene Durchsatz und ein zweiter vorbestimmter Wert verglichen werden, wobei der zweite vorbestimmte Grenzwert einen Methanolverbrauch, der vor der Vorrichtung (100) gemessen wurde, oder einem Methanol-Produktionssollwert entspricht,
- einen Umlaufschritt (350) eines Teils des überschüssigen kalten Syngases am Ausgang des Kondensationsschritts in der ersten Umlaufleitung, die den Ausgang des Kondensationsschritts und den Eingang (310) des Hydrierungsreaktionsschritts (305) verbindet, zu einem Punkt vor dem Messpunkt des Syngasdurchsatzes und
- einen Methanisierungsreaktionsschritt (405), der von einem Methanisierungsreaktor durchgeführt wird, umfassend:
- einen Eingangsschritt (410) für einen Teil des überschüssigen Syngases infolge der Hydrierungsreaktion und nach dem Umlaufschritt (350) eines Teils der kalten Hydrierungsprodukte in der ersten Umlaufleitung und
- einen Ausgangsschritt (415) für Methanierungsreaktionsprodukte, die wenigstens synthetisches Methan enthalten.

10. Verfahren (400) nach Anspruch 9, das aufweist:
- einen Messschritt (420) des Syngasdurchsatzes, der den Eingang des Methanisierungsreaktors durchquert, der vor einer Verbindung zwischen einer zweiten Umlaufleitung, die den Ausgang des Methanisierungsreaktors mit dem Eingang des Methanisierungsreaktors verbindet, positioniert ist, der einen Umlaufkörper, den sogenannten "dritten Umlaufkörper", umfasst,
- einen Steuerschritt (425) des dritten Umlaufkörpers, der so eingerichtet ist, dass er den Umlauf einer Teilmenge von Methanierungsreaktionsprodukten steuert, die in Abhängigkeit von der gemessenen Synthesegasmenge und einem dritten vorbestimmten Sollwert bestimmt wird, und
- einen zweiten Umlaufschritt (430) eines Teils der heißen Methanisierungsreaktionsprodukte in der zweiten Umlaufleitung.

## Claims

1. Device (200) for the cogeneration of methanol and synthetic methane, **characterised in that** it comprises:
- a CO₂ hydrogenation device (100) for producing methanol from a syngas comprising:
- dihydrogen, H₂, output for example from a device for the electrolysis of water, H₂O; and
- carbon dioxide, CO₂,
this hydrogenation device comprising:
- a CO₂ hydrogenation reactor (105) for the production of methanol, referred to as "hydrogenation", comprising:
- an inlet (110) for the syngas; and
- an outlet (115) for hydrogenation reaction products comprising at least methanol, CH₃OH or MeOH,
- a first recirculation pipe (120), for recirculating a portion of the hot hydrogenation reaction products, connecting the reactor outlet to the reactor inlet, the pipe comprising a recirculator (125), referred to as the "first recirculator", of this portion of reaction products;
- a means (130) for measuring the flow rate of the syngas passing through the reactor inlet positioned upstream from a junction between the recirculation pipe and the reactor inlet; and
- a means (135) for controlling the first recirculator configured to compare the flow rate measured with a first predefined value, corresponding to the nominal design flow rate of the hydrogenation reactor (105), as a function of the difference detected, and to control the recirculation of all or part of the stream of hot hydrogenation reaction products in the reactor (105) so that the flow rate at the inlet (110) of this reactor (105) is close to the nominal design flow rate of this reactor (105); and
the hydrogenation device also comprising:
- a condenser (145) of hydrogenation reaction products output from the reactor (105), positioned downstream from a junction between the recirculation pipe (120) and the outlet (115) from the hydrogenation reactor (105), to separate at least the methanol and water from the excess syngas following the hydrogenation reaction;
- a first recirculation line (150), for recirculating a portion of the excess cold syngas on output from the condenser (145), connecting the condenser outlet (155) to the inlet (110) of the hydrogenation reactor (105), upstream from the measurement point measuring the syngas flow rate by the measurement means (130), comprising a recirculator (160), referred to as the "second recirculator", of this syngas;
- a means (165) for measuring the flow rate of the methanol condensed by the condenser (145);
- a means (170) for controlling the second recirculator, configured to compare the flow rate measured with a second predefined value, corresponding to a consumption of methanol measured downstream from the device (100) or to a methanol production setpoint; and
- a methanation reactor (205) downstream from the hydrogenation device comprising:
- an inlet (210) for a portion of the excess syngas following the hydrogenation reaction and downstream from a junction between the condenser outlet and the first recirculation pipe, for recirculating a portion of the excess cold syngas on output from the condenser (145), and
- an outlet (215) for methanation reaction products comprising at least synthetic methane.

2. Device (100) according to claim 1, wherein the hydrogenation device comprises a reaction temperature sensor (180) inside, or on the outlet from, the hydrogenation reactor (105), the syngas inlet (110) of the reactor comprising a heat exchanger (185) whose output temperature is determined as a function of the reaction temperature captured.

3. Device (200) according to one of claims 1 or 2, which comprises:
- a second recirculation pipe (220), for recirculating a portion of the hot methanation reaction products, connecting the methanation reactor outlet to the methanation reactor inlet, the pipe comprising a recirculator (225), referred to as the "third recirculator", of this portion of products;
- a means (230) for measuring the flow rate of the syngas passing through the inlet (210) of the methanation reactor (205) positioned upstream from a junction between the second recirculation pipe and the methanation reactor inlet; and
- a means (235) for controlling the third recirculator configured to control the recirculation of a partial quantity of methanation reaction products determined as a function of the syngas flow rate measured and a predefined third setpoint value (240).

4. Device (200) according to one of claims 1 to 3, which comprises a separator (245) of water contained in the methanation products.

5. Device (200) according to claim 4, which comprises:
- a second recirculation line (250), for recirculating a portion of the cold synthetic methane on output from the separator, connecting the separator outlet to the methanation reactor inlet, the line comprising a recirculator (255), referred to as the "fourth recirculator", of this synthetic methane;
- a means (260) for measuring the methanation reaction temperature inside, or on output from, the methanation reactor (205); and
- a means (265) for controlling the fourth recirculator configured to control the recirculation of a quantity of synthetic methane determined as a function of the temperature (260) measured.

6. Device (200) according to one of claims 1 to 5, which comprises:
- a means (270) for measuring the composition characteristic of the synthetic methane on output from the methanation reactor (205); and
- a means (275) of regulating a flow rate of CO₂ injected into the syngas inlet (110) of the hydrogenation reactor (205), this regulation means being controlled as a function of the characteristic measured and a predefined fourth value (280).

7. Device (200) according to one of claims 1 to 6, which comprises an expander (285) of the syngas between the hydrogenation device (100) and the inlet (210) of the methanation reactor (205) or downstream from the methanation reactor (205).

8. Device (200) according to claim 7, which comprises, upstream from the expander (285), a heat exchanger (290) whose output temperature is determined as a function of a temperature of the syngas measured, by a temperature measurement means (295), downstream from the expander.

9. Method (400) for the cogeneration of methanol and synthetic methane, implemented by a device according to one of claims 1 to 8, **characterised in that** it comprises:
- a CO₂ hydrogenation method (300) for producing methanol from a syngas comprising:
- dihydrogen, H₂, output for example from a step of the electrolysis of water, H₂O; and
- carbon dioxide, CO₂,
this hydrogenation method comprising:
- a CO₂ hydrogenation reaction step (305) for producing methanol, referred to as "hydrogenation", in a hydrogenation reactor, comprising:
- a step (310) of inputting the syngas; and
- a step (315) of outputting the hydrogenation reaction products comprising at least methanol, CH₃OH or MeOH,
- a step (320) of measuring the flow rate of the syngas passing through the inlet of the reactor upstream from a junction between a recirculation pipe, connecting the reactor outlet to the reactor inlet, in a line pipe comprising a recirculator, referred to as the "first recirculator", of a portion of the hot hydrogenation reaction products close to the nominal design flow rate of the hydrogenation reactor;
- a step (325) of controlling the first recirculator configured to control the recirculation of a partial quantity of hydrogenation reaction products determined, wherein the flow rate measured and a first predefined value, corresponding to the nominal design flow rate of the hydrogenation reactor (105), are measured and as a function of the difference detected, and wherein the recirculation of all or part of the stream of hot hydrogenation reaction products in the reactor (105) is controlled so that the flow rate at the inlet (110) of this reactor (105) is close to the nominal design flow rate of this reactor (105); and
the hydrogenation method also comprising:
- a step (335) of condensing hydrogenation products for condensing and separating at least the methanol and water from the excess syngas following the hydrogenation reaction;
- a step (340) of measuring the flow rate of the methanol condensed during the condensing step (335);
- a step (345) of controlling a second recirculator, associated to a first recirculation line, for controlling the recirculation of a partial quantity of excess cold hydrogenation reaction products, not condensed, wherein the flow rate measured and a second predefined value are compared, the second predefined limit value corresponding to a consumption of methanol measured downstream from the device (100) or to a methanol production setpoint;
- a step (350) of recirculating a portion of the excess cold syngas on output from the condensation step, in the first line, connecting the output from the condensation step and the input (310) to the hydrogenation reaction step (305), towards a point located upstream from the measurement point measuring the syngas flow rate, and
- a methanation reaction step (405), performed by a methanation reactor, comprising:
- a step (410) of inputting a portion of the excess syngas following the hydrogenation reaction and after the step (350) of recirculating a portion of the hydrogenation reaction products in the first line; and
- a step (415) of outputting methanation reaction products comprising at least synthetic methane.

10. Method (400) according to claim 9, which comprises:
- a step (420) of measuring the flow rate of the syngas passing through the inlet of the methanation reactor positioned upstream from a junction between the second recirculation pipe connecting the methanation reactor outlet to the methanation reactor inlet, comprising a recirculator, referred to as the "third recirculator";
- a step (425) of controlling the third recirculator configured to control the recirculation of a partial quantity of methanation reaction products determined as a function of the syngas flow rate measured and a predefined third setpoint value; and
- a second step (430) of recirculating a portion of the hot methanation reaction products in the second recirculation pipe.
